(19) Europäisches Patentamt
European Patent Office
Office européen des brevets

(11) EP 2 468 249 A1

(12) **EUROPEAN PATENT APPLICATION**
published in accordance with Art. 153(4) EPC

(43) Date of publication:
**27.06.2012 Bulletin 2012/26**

(21) Application number: **10810022.3**

(22) Date of filing: **20.08.2010**

(51) Int Cl.:
***A61K 8/60*** *(2006.01)* ***A61K 8/02*** *(2006.01)*
***A61K 8/81*** *(2006.01)* ***A61K 8/86*** *(2006.01)*
***A61Q 5/06*** *(2006.01)*

(86) International application number:
**PCT/JP2010/064049**

(87) International publication number:
**WO 2011/021681 (24.02.2011 Gazette 2011/08)**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **20.08.2009 JP 2009191486**

(71) Applicant: **Shiseido Company, Ltd.**
**Chuo-ku**
**Tokyo 104-8010 (JP)**

(72) Inventors:
- **TOYODA, Tomonori**
  **Yokohama-shi**
  **Kanagawa 224-8558 (JP)**
- **FUJIYAMA, Taizo**
  **Yokohama-shi**
  **Kanagawa 224-8558 (JP)**
- **KURASHIMA, Takumi**
  **Yokohama-shi**
  **Kanagawa 224-8558 (JP)**

(74) Representative: **Henkel, Breuer & Partner**
**Patentanwälte**
**Maximiliansplatz 21**
**80333 München (DE)**

(54) **HAIR-DRESSING COSMETIC**

(57) Disclosed is a hair-styling cosmetic which exerts good hair-styling effect and hair-restyling effect, although being water-based and having a low viscosity, shows no stickiness and high smoothness and gives the sense of a favorable light finish. Specifically disclosed is a hair-styling cosmetic containing (a) 0.1-10 mass% of an adhesive resin, and (b) 3-30 mass% of one or more kinds of components selected from among a sugar alcohol, a polyalkylene glycol and derivatives thereof, **characterized in that** said adhesive resin (a) has such properties that a film of 0.1 mm in thickness that is formed of a composition containing said resin substantially at a ratio of 50 mass% has, after drying for 1 day at 25˚C and humidity 50%, an adhesiveness of showing a ball number of 1-30 in the inclined ball tack test (inclination angle 30˚, measured at 25˚C, humidity 50%) . Thus, a hair-styling cosmetic, which exerts good hair-styling effect and hair-restyling effect, shows no stickiness and high smoothness, gives the sense of a favorable light finish, and is suitable for application by mist-spraying, can be obtained.

[Figure 1]

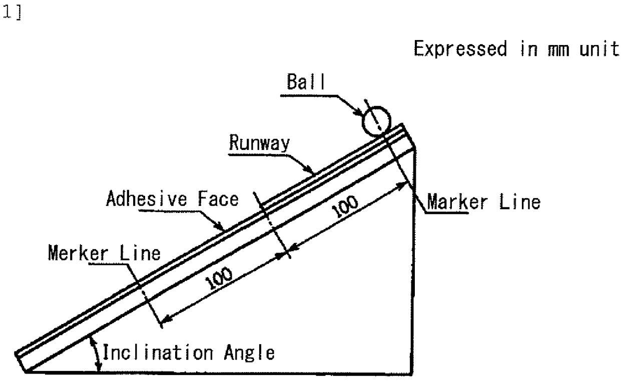




Printed by Jouve, 75001 PARIS (FR)

## Description

TECHNICAL FIELD

The present invention relates to a hair-styling cosmetic. More specifically, the present invention relates to a hair-styling cosmetic that is excellent in hair-styling effect and hair-restyling effect although having a low viscosity, shows no stickiness, shows smoothness, and gives excellent light finish.

BACKGROUND ART

Conventionally, hair setting resins such as a hair fixing polymer and a coating-formable polymer are incorporated for styling in hair-styling cosmetics. However, hair setting resins have defects that they provide stiffness, unevenness of a coating, decrease in styling keeping effect under a high humidity, and the like. Therefore, in order to eliminate the defects, various countermeasures have been adopted.

For example, JP-A-2007-217314 (Patent Document 1) describes that a spray-form powder cosmetic comprising a hair fixing polymer compound, a polyhydric alcohol, a monohydric alcohol and a propellant by respective specific amounts is excellent in hair-restyling property, gives no sticky feeling and has natural glossy feeling.

JP-A-Hll-100312 (Patent Document 2) describes that a hair cosmetic comprising a specific low viscosity polyether compound and a specific polymer resin compound by respective specific amounts has hair-styling effect and hair-styling keeping effect, gives no stickiness and stiffness, and enables hair-restyling by passing the fingers through hair even after drying.

JP-A-H3-261713 (Patent Document 3) describes that a hair cosmetic comprising a specific polyoxyalkylene-based compound and/or polyoxyalkylenealkyl glycoside, a specific hair fixing polymer compound and a specific high molecular weight polyethylene glycol (molecular weight: 6,000 to 30,000) has hair-styling property and smoothing property (smoothness).

JP-A-2002-167317 (Patent Document 4) describes that a hair cosmetic composition comprising an amphoteric polymer, a sugar alcohol and a sugar alcohol derivative (for example, a polyoxyalkylene-adduct of a sugar alcohol, and the like) has hair-styling effect and set keeping effect, and gives no sticky feeling and stiff feeling.

JP-A-2004-505902 (Patent Document 5) describes that a hair-care composition comprising a specific water-soluble polyalkylene glycol and a specific coating-forming polymer in a specific ratio and further comprising a liquid carrier is excellent in re-styling property and feeling.

However, the above-mentioned publications specifically fail to describe or suggest the objects or problems to obtain hair-styling effect and hair-restyling effect that can be satisfied sufficiently in a water-based low viscosity hair-styling cosmetic, and to obtain a hair-styling cosmetic that exhibits effects that the cosmetic gives no sticky feeling, is smooth and gives excellent light finish. In a water-based hair-styling cosmetic having a low viscosity, it is especially difficult to balance setting effect and arrangement effect. Therefore, development of a water-based low viscosity hair-styling cosmetic that has sufficient setting effect and arrangement effect in combination and also has fine feeing of use has been required.

PRIOR ART DOCUMENTS

PATENT DOCUMENTS

Patent Document 1: JP-A-2007-217314

Patent Document 2: JP-A-Hll-100312

Patent Document 3: JP-A-H3-261713

Patent Document 4: JP-A-2002-167317

Patent Document 5: JP-A-2004-505902

## SUMMARY OF INVENTION

## PROBLEM TO BE SOLVED BY THE INVENTION

The present invention has been made in view of the above-mentioned conventional situation, and aims at providing a hair-styling cosmetic that is excellent in hair-styling effect and hair-restyling effect although being an aqueous-based and having a low viscosity, shows no stickiness, shows smoothness and gives excellent light finish.

## MEANS FOR SOLVING PROBLEM

The present inventors have done intensive studies, and consequently found that the above-mentioned problem is solved by incorporating an adhesive resin having predetermined adhesiveness and one kind or two kinds or more selected from sugar alcohols, polyalkylene glycols and derivatives thereof by predetermined amounts, and completed the present invention. Namely, the present invention provides a hair-styling cosmetic comprising (a) 0 . 1 to 10% by mass of an adhesive resin having such an adhesiveness as showing a ball number of from 1 to 30 in an inclined ball tack test (inclination angle: 30˚, 25˚C, humidity: 50%), and (b) 3 to 30% by mass of one kind or two kinds or more selected from sugar alcohols, polyalkylene glycols and derivatives thereof.

## EFFECT OF THE INVENTION

Since the hair-styling cosmetic of the present invention incorporate the adhesive resin and the specific components: one kind or two kinds or more selected from sugar alcohols, polyalkylene glycols and derivatives thereof by predetermined amounts and is prepared so as to have a low viscosity, it is suitable for use by atomizing into a form of mist, and can exhibit excellent hair-styling effect and hair-restyling effect.

## BRIEF DESCRIPTION OF DRAWINGS

FIG. 1 is a schematic view showing an apparatus for a ball tack test.

## MODE FOR CARRYING OUT THE INVENTION

The hair-styling cosmetic of the present invention comprises an adhesive resin having predetermined adhesiveness (component a) as an essential component.
In the present specification, the adhesiveness of the adhesive resin is specified by a ball tack test according to JIS Z 0237. The ball tack test is a method for testing adhesiveness comprising measuring by using a measurement apparatus as shown in FIG. 1. Specifically, when a sample to be measured ( adhesive material) is disposed on the inclined plane of the measurement apparatus of FIG. 1, the part from the top of the inclined plane to the position at 10 cm from the top is covered with a non-adhesive sheet to form an entrance path, and balls each made of a predetermined material and having a predetermined size are each rolled from the top of the inclined plane, the adhesiveness is specified by the number of the ball having the maximum size among the balls that have stopped on either position of the adhesive surface.
Specifically, in the adhesive resin used in the present invention, a ball tack test was conducted by using a sample surface that was obtained by preparing a composition substantially comprising the resin by 50% by mass, making a coating having a thickness of 0.1 mm on the inclined surface of FIG. 1 by using the composition, and drying the coating at 25˚C and a humidity of 50% for 1 day. The measurement was conducted under the measurement conditions of an inclination angle of 30˚, 25˚C and a humidity of 50% in the measurement apparatus shown in FIG. 1, and the resin having such a adhesiveness as showing a ball number of from 1 to 30, more preferably from 3 to 25 was used as the adhesive resin in the present invention.
Specific examples of the adhesive resin used in the present invention can include acrylic-based resins such as Acronal YJ2720D, Acronal V215ap, Acronal N285, Acronal V210 and Acronal V212 (manufactured by BASF), NCOR38-088A, Duro-Tak87-200A and Duro-Tak387-2054/87-2054 (manufactured by National Starch & Chemical), MG-0560 and MG-0580 (manufactured by Dow Corning), Daitosol 5000AD that is an alkyl acrylate ester (manufactured by Daito Kasei Kogyo Co. , Ltd.), and Yodozol AH955 and PRIMALPS83-D that belong to alkyl methacrylate ester/alkyl acrylate ester copolymers (manufactured by Rohm & HAAS), and the like.
Alternatively, as the adhesive resin used in the present invention, a adhesive setting resin obtained by polymerizing the following monomer A and/or monomer B and monomer C and monomer D may be selected.

Monomer A: At least one kind of monomer represented by the following formula:

[Chemical formula 1]

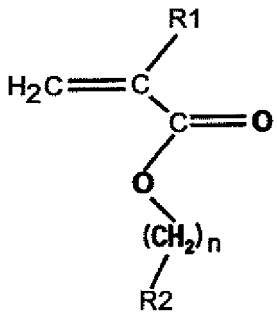

(A)

wherein R1 is H or $CH_3$, n is an integer of from 0 to 30, $(CH_2)_n$ comprises branched chain (s), and R2 is H, OH, $OCH_3$, $OCH_2CH_3$ or phenyl.

Monomer B: At least one kind of monomer represented by the following formula:

[Chemical formula 2]

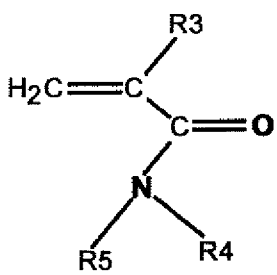

(B)

wherein R3 is H or CH3, R4 and R5 may be the same of different and are each H or $(CH_2)_1R'$ wherein I is an integer of from I to 3 and R' is H, OH or -NR"R"' wherein R" and R"' may be the same of different and are each H or a C1-C3 alkyl group.

Monomer C: At least one kind of monomer represented by the following formula:

[Chemical formula 3]

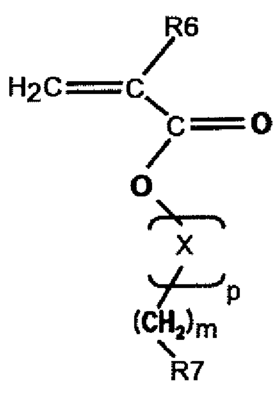

(C)

wherein R6 is H or $CH_3$, p is an integer of from I to 100, m is an integer of from 0 to 30, R7 is H, OH, $OCH_3$, $OCH_2CH_3$ or phenyl, and X is an oxyethylene group (EO), an oxypropylene group (PO), an oxybutylene group (BO) or glyceryl.

Monomer D: At least one kind of monomer represented by the following formula:

[Chemical formula 4]

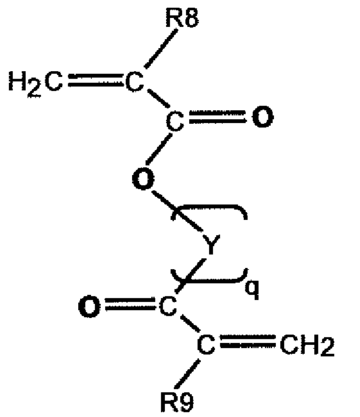

(D)

wherein R8 is H or $CH_3$, q is an integer of from 1 to 100, Y is an oxyethylene group (EO), an oxypropylene group (PO), an oxybutylene group (BO), a straight chain or branched oxyalkylene group having 5 or more carbon atoms or glyceryl, provided that q is 1 when Y is an oxyalkylene group having 5 or more carbon atoms.

As the adhesive setting resin used in the present invention, one having a structure represented by the following formula (I) is specifically preferable.

[Chemical formula 5]

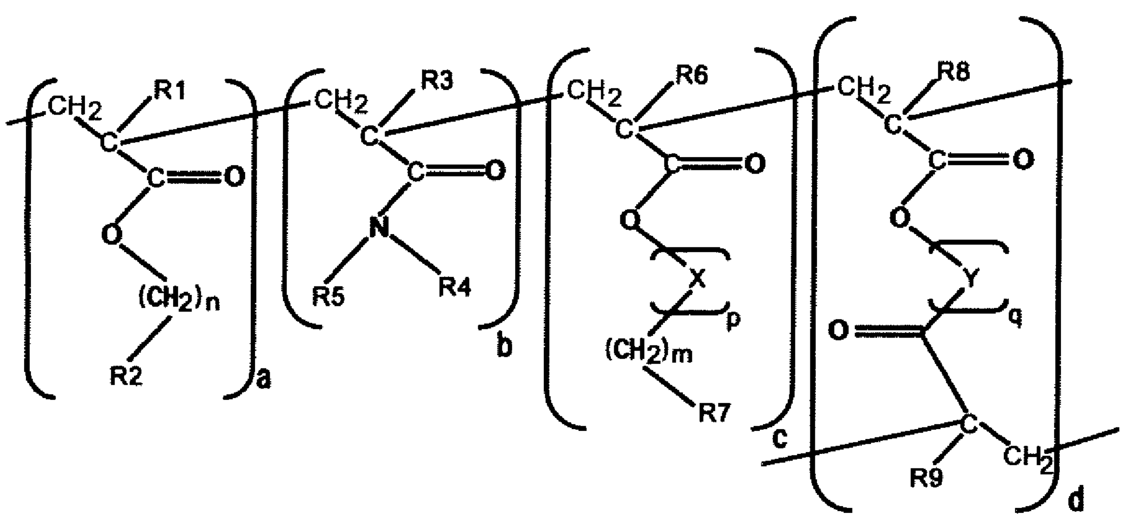

(I)

In the above-mentioned formula (I), R1 to R9, n, m, p and q have the similar meanings as in the above-mentioned formulas A to D. The terms in the present specification are used in general meanings; for example, the oxyethylene group (EO), oxypropylene group (PO) and oxybutylene group (BO) mean straight chain or branched oxyalkylene groups having 2, 3 and 4 carbon atoms, respectively. Furthermore, in the above-mentioned formula (I), a is a number within the range of $40<a<400$, b is a number within the range of $80\leq b<300$, c is a number within the range of $30<c<300$ and d is a number within the range of $0<d<10$.

The mass percents of the respective monomers in the adhesive setting resin (the polymer of the formula (I)) that satisfy

the above-mentioned conditions are approximately as follows. 7.5<A<62.5, 20≤B<45, 7.5<C<60, 0<D<5.

The adhesive setting resin used in the invention can be prepared by mixing the above-mentioned monomer A and/or B and C and D in a suitable ratio and subjecting the mixture to a polymerization reaction by using a general method, in a suitable solvent as necessary. For example, it can be obtained by heat-polymerizing at about 80˚C for 8 hours by using a polymerization initiator such as 2,2'-azobisisobutyronitrile in ethanol.

As a silicone-based adhesive resin, addition reaction-type silicones such as SD4584, SD4560, SD4570, SD4580, SD4585 and SD4587L (manufactured by Dow Corning Toray Silicone Company Ltd.), and TSR1512 and TSR1516 (manufactured by MOMENTIVE) can be given.

In the present invention, the resin is not specifically limited to the specific examples listed above as long as it is a resin that satisfies the above-mentioned conditions of adhesiveness, and one kind or two kinds or more of those can be mixed and used.

The incorporation amount of the adhesive resin in the cosmetic of the present invention is from 0 . 1 to 10% by mass, more preferably from 0.5 to 7.5% by mass. When the incorporation amount is less than 0.1% by mass, a desired effect may not be obtained, and when the incorporation amount exceeds 10% by mass, stickiness is caused.

The hair-styling cosmetic of the present invention comprises one kind or two kinds or more selected from sugar alcohols, polyalkylene glycols and derivatives thereof (component b) in addition to the adhesive resin, as an essential component.

The sugar alcohol used in the present invention is a polyhydric alcohol obtained by reducing the carbonyl group of a sugar. Specifically, maltitol ("Malbit"; manufactured by B Food Science Co., Ltd.), sorbitol ("Sorbitol C"; manufactured by B Food Science), ribitol, mannitol, arabitol, galactitol, xylitol, erythritol, inositol and the like can be exemplified. Alternatively, a derivative of a sugar alcohol can also be used in the present invention. For example, POE-POP-added (oleic acid polyoxyethylene sorbit "Rheodol 430V": Kao Chemicals, polyoxypropylene sorbit "Uniol HS-1600D"), alkyl group-added, cationized, anionized and silylated derivatives, and the like can be exemplified.

Among these, sorbitol and maltitol are preferable from the viewpoints of absence of stickiness and stiffness, and the like.

Preferable examples of the polyalkylene glycols and derivative thereof used in the present invention may include EO polymers in which ethylene oxide (EO) constitutional units have been polymerized, PO polymers in which propylene oxide (PO) constitutional units have been polymerized, BO polymers in which butylene oxide (BO) constitutional units have been polymerized, and copolymers in which the above-mentioned constitutional units have been copolymerized, and the like. Specifically, EO polymers, EO-PO copolymers comprising EO constitutional units and PO constitutional units, EO-BO copolymers comprising EO constitutional units and BO constitutional units, and the like are preferable. The format of copolymerization is not specifically limited, and is any of block copolymerization, graft copolymerization, random copolymerization and the like.

As the polyalkylene glycols used in the present invention, commercially available ones can be utilized, and examples can include EO-adduct polymers: PEG200, PEG300, PEG400, PEG600, PEGIOOO, PEG1540, PEG2000, PEG4000, PEG6000, PEG11000 and PEG20000 (NOF Corporation or Toho Chemical Industry Co., Ltd.), PO-adduct polymers: Uniol D-700, Uniol D-1000, Uniol D-1200 and Uniol D-2000 (NOF Corporation), and the like.

Although the polyalkylene glycols used in the present invention are not specifically limited, among those, polyalkylene glycols are optimal.

The polyalkylene glycol derivatives used in the present invention encompass the following hair-styling oil component and the like. The hair-styling oil component means an EO/PO adduct of a mono- to tetravalent alcohol or a mono- to trivalent carboxylic acid. Commercially available ones can be utilized, and examples can include Unilub 50MB168, Unilub MB370, Triol G-40, Savondor SGP-7 and Savondor GP-9 (NOF Corporation), and the like.

The incorporation amount of the sugar alcohols, polyalkylene glycols and/or derivatives thereof (component b) in the cosmetic of the present invention is from 3 to 30% by mass, more preferably from 5 to 25% by mass. When the incorporation amount is less than 3% by mass, a desired effect may not be obtained, and when the component is incorporated by going beyond 30% by mass, stickiness and stiffness may be caused.

Since the hair-styling cosmetic of the present invention has a low viscosity, the solvent volatilizes quickly during application to hair, thereby adhesiveness is exhibited. Specifically, it is preferable that the viscosity measured with a B-type viscometer at 25˚C is adjusted to 100 mPa·s or less. The effect of the invention is exerted easily by atomizing into a form of mist. When the viscosity exceeds 100 mPa·s, the cosmetic may become unsuitable for atomizing into a form of mist, for example, problems such as clogging in a nozzle of an atomizing apparatus may be caused.

Furthermore, in the hair-styling cosmetic of the present invention, the range of the adhesiveness after the solvent in the cosmetic has been volatilized is also an important factor. By adjusting the adhesiveness to a predetermined range, stickiness during application to hair can be suppressed, and hair-restyling effect can be improved.

Specifically, it is preferable to adjust the adhesiveness to such an adhesiveness as showing a ball number of from 1 to 30, more preferably from 3 to 25, wherein the ball number is measured at an inclination angle of the inclined plane in the above-mentioned ball tack test (the measurement apparatus shown in FIG. 1) of 10˚ and 25˚C after forming a coating having a thickness of 0.1 mm by using the cosmetic of the present invention and drying at 25˚C and a humidity of 50%.

When the adhesiveness is so low that the ball No. 1 is not stopped, hair-restyling effect is insufficient, whereas when the adhesiveness is so high that a ball larger than the ball No. 30 is stopped, hair may become sticky.

Furthermore, it is preferable that the hair-styling cosmetic of the present invention comprises an oil component by from 0.01 to 5% by mass, more preferably from 0 . 1 to 3% by mass. By comprising the oil component, further smoothness can be imparted to hair.

The oil component used in the present invention is not specifically limited and can be selected from oil components that have been conventionally used in cosmetics, specifically in hair cosmetics. Examples may include hydrocarbon oils such as heavy isoparaffins (=hydrogenated polyisobutenes) and light isoparaffins (for example, Isopar A, C, E, G, H, K, L and M (manufactured by Exxon), Shellsol 71 (manufactured by Shell), Soltrol 100, 130 and 220 (manufactured by Phillips), and the like), squalane and liquid paraffin; esters such as cetyl-2-ethyl hexanoate, 2-ethylhexyl palmitate, 2-octyldodecyl myristate, neopentyl glycol-2-ethylhexanoate, isopropyl myristate, myristyl myristate and tetra 2-ethylhexanoic acid pentaerythrityl; fats such as olive oil, avocado oil, jojoba oil, sunflower seed oil, safflower oil, camellia oil, macadamia nut oil, mink oil, liquid lanolin, lanolin acetate and castor oil; silicone-based oils such as dimethylpolysiloxane, methylphenylpolysiloxane, gum-like dimethylpolysiloxane having a high polymerization degree, polyether-modified silicone, amino-modified silicone, low-boiling point silicone oils (hexamethylcyclotrisiloxane, octamethyltetracyclosiloxane [for example, "Execol D-4" (manufactured by Shin-Etsu Silicone Co., Ltd.), "SH244" and "SH344" (manufactured by Dow Corning Toray Silicone Co., Ltd.) and the like], decamethylcyclepentasiloxane [for example, "Execol D-5" (manufactured by Shin-Etsu Silicone Co., Ltd.), "SH245" and "DC345" (manufactured by Dow Corning Toray Silicone Co., Ltd.), and the like], dodecamethylcyclehexasiloxane [for example, "DC246" (manufactured by Dow Corning Toray Silicone Co., Ltd.)] tetradecamethylcycloheptasiloxane, and the like), and the like; fluorine-based oil components such as fluorine-modified dimethylpolysiloxanes, fluorine-modified methylphenylpolysiloxanes, perfluoropolyethers and perfluorocarbons; and the like.

It is preferable that the hair-styling cosmetic of the present invention comprises water and an alcohol in addition to the above-mentioned adhesive resin and sugar alcohols, polyalkylene glycols and derivatives thereof.

As the alcohol in the hair-styling cosmetic of the present invention, one kind or two kinds or more selected from alcohols that are generally used in cosmetics such as ethanol can be suitably selected and used. The incorporation amounts of the water and alcohol are not specifically limited, and are suitably adjusted within a range in which the above-mentioned predetermined adhesiveness and viscosity are given.

The hair-styling cosmetic of the present invention can be provided in various embodiments such as a hair liquid, a hair foam, a hair mousse, a hair spray, a hair mist, a hair gel and a hair wax, and is specifically suitable for a form for using by atomizing into a form of mist since it is a cosmetic that comprises the adhesive resin and sugar alcohols and/or polyalkylene glycol derivatives by predetermined amounts and may have limited adhesiveness and viscosity. By using in a form of mist, the solvent volatilizes quickly, and adhesiveness and hair-styling effect can be exerted quickly.

The hair-styling cosmetic of the present invention may incorporate, depending on the form thereof, for example, other components that have been conventionally used in hair-styling cosmetics, to the extent that the effect of the invention is not deteriorated.

## EXAMPLES

Hereinafter the present invention will be explained in more detail with referring to specific examples, but the present invention is not construed to be limited to the following Examples. Furthermore, the incorporation amounts in the following Examples and the like are represented by % by mass unless otherwise specified.

(Preparation Examples and Comparative Preparation Examples)

Polymerization was conducted by the monomer compositions shown in the following Table 1 to prepare adhesive setting resins that may be used in the present invention (Preparation Examples 1 to 6), and Comparative Preparation Example 1 that was free from monomer C and Preparation Example 2 that was free from monomer D.

Specifically, a mixture comprising 100 parts of a monomer is prepared in advance; 100 parts of ethanol is charged in a 1 L volume five-necked flask equipped with a dropping funnel containing this mixture, a reflux condenser, a thermometer, a tube for nitrogen replacement and a stirrer; the temperature is raised under nitrogen flow; 1 part of a polymerization initiator (2,2-azobisisobutyronitrile) is added to the ethanol when the ethanol reaches a reflux state (about 80˚C); and the above-mentioned mixture is added dropwise continuously for 2 hours. Thereafter the reactant was left in the reflux state for 8 hours to promote a polymerization reaction. The solvent was then distilled off from the solution in the five-necked flask, and ethanol was added to adjust the solvent content of this solution to give a solution of a cosmetic base having a solid content concentration of 50%.

[Table 1]

| Classification | Chemical structure (trade name) | Manufacturer | Preparation Example 1 | Preparation Example 2 | Preparation Example 3 | Comparative Preparation Example 2 | Preparation Example 4 | Preparation Example 5 | Preparation Example 6 | Comparative Preparation Example 1 |
|---|---|---|---|---|---|---|---|---|---|---|
| A | Idemitsu Butyl acrylate | Kosan Co., Ltd . | 40 | 35 | 30 | 40 | 15 | | | |
| A | Ethyl acrylate | Toagosei Co., Ltd. | | | | | | 30 | | 30 |
| A | Stearyl methacrylate (Blenmer SMAI | NOF Corporation | | | | | | | | |
| A | Hydroxyethyl acrylate (HEA) | Osaka Organic Chemical Industry | | | | Ltd. | 15 | | | 30 |
| A | Methoxyethyl acrylate (Aronix C-1) | Toagosei Co., Ltd. | | | | | | 15 | | |
| B | Dimethyacrylamide Dimethyacrylanide (DMAA) Dimethyacrylamide | Kohjin Chemical Co., Ltd. Co., Ltd. | | 40 | 30 | 30 | 30 | | 40 | 35 |
| B | Dimethyaminopropylacrylamide (DMAPAA) | Toagosei Co., Ltd. | | | | 20 | | 20 | | |
| C | Acrylic acid polyoxyethylene glycol (n=10) (Blenmer AE-400 | NOF corporation | 55 | 20 | 15 | 30 | 15 | | | |
| C | Acrylic acid polyoxypropylene glycol (n=6) (Blenmer AP-400) | NOF Corporation | | | 20 | | 20 | 30 | 55 | |
| D | Diacrylic acid polyoxyethylene glycol (n=23) (NE Ester A-1000) | Shin-Nakamura Chemical Co., Ltd. | 5 | 5 | 5 | | 5 | | | 5 |

(continued)

| Classification | Chemical structure (trade name) | Manufacturer | Preparation Example 1 | Preparation Example 2 | Preparation Example 3 | Comparative Preparation Example 2 | Preparation Example 4 | Preparation Example 5 | Preparation Example 6 | Comparative Preparation Example 1 |
|---|---|---|---|---|---|---|---|---|---|---|
| D | Dimethacrylic acid polyoxyethyleneglycol (n=14) (NK Ester 14G) | Shin-Nakamura Chemical Co., Ltd. | | | | | | | 5 | |
| D | Glycerin dimethacrylate (Blenmer NDMA) | NOF Corporation | | | | | | 5 | | |

(Examples and Comparative Examples)

For the adhesive resins in the following Table 2, the adhesiveness thereof was measured by a ball tack test. In the tables, the result of the ball tack test represented by "-" shows that the adhesiveness was so low that the ball No. 1 did not stop.

[Table 2]

| adhesive resin | | Ball tack test (30˚, 25˚C, humidity 50%) |
|---|---|---|
| Type (manufacturer) | Trade name | |
| Acrylic-based (BASF) | Acronal YJ2720D | 1 |
| Acrylic-based (BASF) | Acronal V215ap | 12 |
| Acrylic acid alkyl ester | Daitosol 5000AD | 4 |
| Methacrylic acid alkyl ester | n-Butyl acrylate/2-ethylhexyl acrylate/ methyl methacrylate copolymer emulsion {*1} | 1 |
| Silicone-based (Dow Corning Toray) | SD4584PSA | 4 |
| adhesive setting resin of Preparation Example 3 | - | 5 |
| Vinyl acetate | S-Dine 5301 | - |
| n-Butyl acrylate/2-ethylhexyl acrylate/methyl methacrylate copolymer emulsion (manufactured by Daito Kasei Kogyo Co., Ltd.): Resin solid content:water=l:l (mass ratio) in this resin emulsion. | | |

Next, samples having the compositions as listed in the following Tables 3 and 4 were prepared, the arrangement effect, adhesion effect, fixing effect, hair-restyling effect and absence of feeling of dryness when the samples were used were evaluated. The results thereof are also shown in Tables 3 and 4. The evaluation methods and evaluation criteria for the respective properties are as follows.

1. Arrangement effect

0.5 g of the sample was applied to a bundle of black virgin hair (length: 20 cm, mass: 2 g) and dried at an ordinary temperature, and the obtained hair bundle was evaluated for easiness of arrangement by a sensory test by specialized panelists.

<Criteria for evaluation points>

5 points: Arrangement is considerably easy
4 points: Arrangement is slightly easy
3 points: Normal
2 points: Arrangement is slightly difficult
1 point: Arrangement is difficult

<Evaluation criteria>

⊙: The total points are 40 points or more
O: The total points are 30 points or more and less than 40 points
△: The total points are 20 points or more and less than 30 points
×: The total points are less than 20 points

2. Fixing effect

0.4 g of the sample was applied to black virgin hair (length: 15 cm, weight: 1 g) and spreaded by using a comb,

the shape of the hair was arranged so that the hair was relaxed, and 5 strands were prepared for one sample. The strand was dried for 1 hour at 50˚C and hanged on a scaled board, and the flexure length (b) of the strand was measured in a constant temperature and humidity chamber at a temperature of 30˚C and a humidity of 90% RH. Using the flexure length (a) of the strand that had been measured in advance before applying the sample, fixing effect (keeping effect) was obtained according to the following equation. A numerical value closer to 100% represents higher fixing effect and more excellent humidity resistance.

$$\text{Hair style keeping effect (\%)} = \{(a-b)/a\} \times 100$$

<Evaluation criteria>

⊙: The value is 90% or more
○: The value is 70% or more and less than 90%
△: The value is 50% or more and less than 70%
×: The value is less than 50%

3. Hair-restyling effect

0.5 g of the sample was applied to a bundle of black virgin hair (length: 20 cm, mass: 2 g) and dried at an ordinary temperature, and the obtained hair bundle was evaluated for easiness of hair styling when the hair was trimmed immediately after the application and trimmed again after 1 hour (hair-restyling effect) by a sensory test by specialized panelists.

<Criteria for evaluation points>

5 points: Hair-restyling effect is considerably high
4 points: Hair-restyling effect is slightly high
3 points: Normal
2 points: Hair-restyling effect is slightly low
1 point: Hair-restyling effect is not exhibited

<Evaluation criteria>

⊙: The total points are 40 points or more
○: The total points are 30 points or more and less than 40 points
△: The total points are 20 points or more and less than 30 points
×: The total points are less than 20 points

3. Adhesion effect

0.5 g of the sample was applied to a bundle of black virgin hair (length: 20 cm, mass: 2 g) and dried at an ordinary temperature, and the obtained hair bundle was evaluated for adhesion effect on the hair by a sensory test by specialized panelists.

<Criteria for evaluation points>

5 points: Adhesion is felt considerably
4 points: Adhesion is felt slightly
3 points: Normal
2 points: Adhesion is felt slightly little
1 point: Adhesion is not felt

<Evaluation criteria>

⊙: Total points are 40 points or more
○: Total points are 30 points or more and less than 40 points
△: Total points are 20 points or more and less than 30 points
×: Total points are less than 20 points

5. Absence of feeling of dryness

0.5 g of the sample was applied to a bundle of black virgin hair (length: 20 cm, mass: 2 g) and dried at an ordinary temperature, and the obtained hair bundle was evaluated for the absence of feeling of dryness on hair during trimming by a sensory test by specialized panelists.

<Criteria for evaluation points>

5 points: Feeling of dryness is considerably little
4 points: Feeling of dryness is slightly little
3 points: Normal
2 points: Dryness is slightly felt
1 point: Dryness is felt

<Evaluation criteria>

⊙: Total points are 40 points or more
○: Total points are 30 points or more and less than 40 points
△: Total points are 20 points or more and less than 30 points
×: Total points are less than 20 points

6. Humidity resistance

0.5 g of the sample (the hair cosmetic obtained in Example or Comparative Example) was applied to black virgin hair (length: 20 cm, weight: 2 g), and a curl was immediately made by using a curler having a curl diameter of 2 cm and dried at 50˚C for 1 hour. The length of the curled hair strand was measured, and said length was defined as an initial value (L0) .
Next, the dried hair bundle was hanged on a scaled board and put into a constant temperature and humidity chamber at a temperature of 30˚C and a humidity of 90%RH for 3 hours, and the length of the hair strand was measured and defined as strength after humidification (L2).
The length of the hair strand is the maximum diameter when the hair is in a curled state, whereas the length is the maximum length from the end part at the base side (for example, the length from the end part at the base side to the end part of the tip) when the curl is uncoiled partially or entirely.
The curl retention value was calculated according to the following equation, and the humidity resistance was evaluated according to the following evaluation criteria.

$$\text{Curl retention value (\%)} = \{(20-L2)/(20-L0)\} \times 100$$

A curl retention value closer to 100% represents a stronger curl keeping rate and more excellent humidity resistance (i.e., style keeping effect).

<Evaluation criteria>

⊙: The curl retention value is 90% or more.
○: The curl retention value is 70% or more and less than 90%
△: The curl retention value is 50% or more and less than 70%
×: The curl retention value is less than 50%

[Table 3]

| | Comparpative Example 1 | Comparative Example 2 | Comparative Example 3 | Comparative Example 4 | Comparative Example 5 | Example 1 | Example 2 | Example 3 | Example 4 | Example 5 | Example 6 |
|---|---|---|---|---|---|---|---|---|---|---|---|
| Water | Amount that makes the entirety 100% | | | | | | | | | | |
| Ethanol | 5 | 5 | 5 | 5 | 5 | 5 | 5 | 5 | 5 | 5 | 5 |
| Acronal V215ap | | 10 | | | | 10 | | 10 | | 10 | |
| Daitosol 5000AD | | | 10 | | | | 10 | | 10 | | 10 |
| Multitol | | | | 5 | | 5 | 5 | | | 5 | 5 |
| PEG-6 | | | | | 2.5 | | | 2.5 | 2.5 | 2.5 | 2.5 |
| PEG-32 | | | | | 2.5 | | | 2.5 | 2.5 | 2.5 | 2.5 |
| | | | | | | | | | | | |
| Total | 100 | 100 | 100 | 100 | 100 | 100 | 100 | 100 | 100 | 100 | 100 |
| | | | | | | | | | | | |
| Fixing effect | × | Δ | Δ | × | × | ○ | ○ | ○ | ○ | ○ | ○ |
| Adhesion effect | × | ○ | ○ | ○ | ○ | ⊙ | ⊙ | ⊙ | ⊙ | ⊙ | ⊙ |
| Arranging effect | × | ○ | ○ | ○ | Δ | ⊙ | ○ | ⊙ | ○ | ⊙ | ⊙ |
| Hair-restyli ng effect | × | ○ | ○ | Δ | Δ | ○ | ○ | ○ | ○ | ⊙ | ○ |
| Absence of feeling of dryness | × | × | × | Δ | ○ | ○ | ○ | ○ | ○ | ⊙ | ⊙ |

[Table 4]

| | Example 7 | Example 8 |
|---|---|---|
| Ion exchanged water | Amount that makes the entirety 100% | |
| Ethanol | 50 | 50 |
| Sorbitol | - | 1 |
| PEG-6 | 5 | 2 |
| PEG-8 | 5 | 3 |
| PEG-32 | 5 | 2 |
| (Alkyl acrylate/diacetoneacrylamide)copolymer AMP (*2) | 3 | 1 |
| Polyacrylate cross polymer-3 | 2 | 1 |
| Citric acid | Suitable amount | Suitable amount |
| Phenoxyethanol | Suitable amount | Suitable amount |
| Fragrance | Suitable amount | Suitable amount |
| Ball tack test (Inclination angle 10˚, 25˚C) | 5 | 3 |
| | | |
| Fixing effect | ○ | ○ |
| Arranging effect | ⊙ | ⊙ |
| Hair-restyling effect | ⊙ | ○ |
| Humidity resistance | ○ | ○ |
| (*2) PLAS CIZE (manufactured by Goo Chemical Co., Ltd.) | | |

Other examples are shown below.

(Example 9)

| | |
|---|---|
| Liquid-type styling agent | |
| (1) Ion exchanged water | balance |
| (2) PEG-6 | 7 |
| (3) PEG-32 | 7 |
| (4) Maltitol | 10 |
| (5) PEG/PPG-55/28 dimethyl ether | 1 |
| (6) Ethanol | 5 |
| (7) Fragrance | suitable amount |
| (8) Acronal YJ2720D | 1 |
| (9) (Alkyl acrylate/diacetoneacrylamide) copolymer AMP | 2 |
| (10) Citric acid | suitable amount |
| (11) Phenoxyethanol | suitable amount |
| (12) PEG-40-butyl | 1 |

<Preparation method>

The water-soluble components (2) to (5) were added to the water (1) and dissolved by stirring to give a water

part. Next, (7) was added to the ethanol (6) and solubilized by stirring, and (5), (9) and (11) were added thereto and stirred to give an alcohol part. The water part and alcohol part were mixed, and (8), (10) and (12) were added thereto to give a liquid-type styling agent.

(Example 10)

| Liquid-type styling agent | |
|---|---|
| (1) Ion exchanged water | balance |
| (2) PEG-6 | 5 |
| (3) PEG-8 | 5 |
| (4) PEG-32 | 5 |
| (5) Sorbitol | 1 |
| (6) Ethanol | 50 |
| (7) Fragrance | suitable amount |
| (8) Adhesive setting resin of Preparation Example 3 | 3 |
| (9) (Alkyl acrylate/diacetoneacrylamide) copolymer | 2 |
| (10) Citric acid | suitable amount |

<Preparation method>

The water-soluble components (2) to (5) were added to water (1) and dissolved by stirring to give a water part. Next, (7) was added to (6) and solubilized by stirring, and (8) to (9) were added thereto and stirred to give an alcohol part. The water part and alcohol part were mixed, and (10) was added thereto to give a liquid-type styling agent.

(Example 11)

| Liquid-type styling agent | |
|---|---|
| (1) Ion exchanged water | balance |
| (2) PEG-6 | 7 |
| (3) PEG-32 | 7 |
| (4) Maltitol | 10 |
| (5) Ethanol | 10 |
| (6) Fragrance | suitable amount |
| (7) PEG-60 hydrogenated castor oil | 0.5 |
| (8) Hydrogenated polyisobutene | 0.3 |
| (9) Acronal V215ap | 1 |
| (10) (Alkyl acrylate/diacetoneacrylamide) copolymer | 2 |
| (11) Phenoxyethanol | suitable amount |
| (12) Citric acid | suitable amount |

<Preparation method>

The water-soluble components (2) to (4) were added to water (1) and dissolved by stirring to give a water part. Next, (6), (7) and (8) were added to ethanol (5) and solubilized by stirring, and (10) and (11) were added thereto and stirred to give an alcohol part. The water part and alcohol part were mixed, and (9) and (12) were added thereto to give a liquid-type styling agent.

**Claims**

1. A hair-styling cosmetic, comprising:

   (a) 0.1 to 10% by mass of an adhesive resin, and
   (b) 3 to 30% by mass of one kind or two kinds or more selected from sugar alcohols, polyalkylene glycols and derivatives thereof,

   wherein the adhesive resin (a) has such a adhesiveness as showing a ball number of from 1 to 30 in an inclined ball tack test (inclination angle: 30˚, 25˚C, humidity: 50%) after forming a coating having a thickness of 0.1 mm by using a composition substantially comprising the resin by 50% by mass and drying at 25˚C and a humidity of 50% for 1 day.

2. The hair-styling cosmetic according to claim 1,
   wherein the cosmetic has a viscosity of 100 mPa·s or less (25˚C, a B-type viscometer).

3. The hair-styling cosmetic according to claim 1 or 2,
   wherein the cosmetic has such a adhesiveness as showing a ball number of from 1 to 30 in an inclined ball tack test (inclination angle: 10˚, 25˚C, humidity: 50%) after forming a coating having a thickness of 0.1 mm by using the cosmetic and drying at 25˚C and a humidity of 50% for 1 day.

4. The hair-styling cosmetic according to any one of claims 1 to 3, further comprising:

   0.01 to 5% by mass of an oil component.

5. The hair-styling cosmetic according to any one of claims 1 to 4,
   wherein the hair-styling cosmetic is used by atomizing into a form of mist.

[Figure 1]

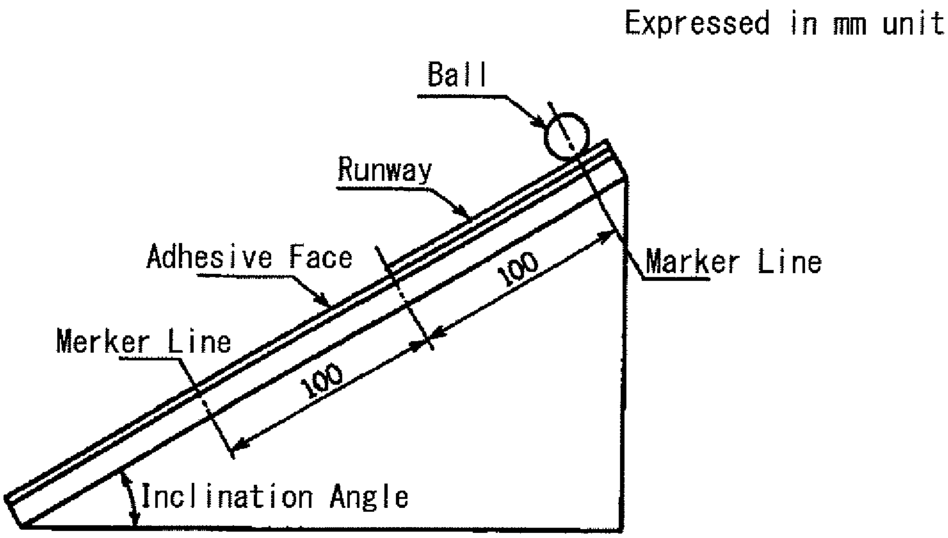
Expressed in mm unit
Ball
Runway
Adhesive Face
Merker Line
Marker Line
100
100
Inclination Angle

## INTERNATIONAL SEARCH REPORT

International application No. PCT/JP2010/064049

A. CLASSIFICATION OF SUBJECT MATTER
*A61K8/60*(2006.01)i, *A61K8/02*(2006.01)i, *A61K8/81*(2006.01)i, *A61K8/86*(2006.01)i, *A61Q5/06*(2006.01)i

According to International Patent Classification (IPC) or to both national classification and IPC

B. FIELDS SEARCHED

Minimum documentation searched (classification system followed by classification symbols)
A61K8/60, A61K8/02, A61K8/81, A61K8/86, A61Q5/06

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2010 |
| Kokai Jitsuyo Shinan Koho | 1971-2010 | Toroku Jitsuyo Shinan Koho | 1994-2010 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | JP 2009-520708 A (Unilever N.V.), 28 May 2009 (28.05.2009), claims 1, 8; paragraphs [0012] to [0014], [0018] to [0020], [0026], [0027], [0057]; tables 1, 2, 4; examples 6, 8, 9 & EP 1965759 A1 & WO 2007/071308 A1 | 1-5 |
| P,A | JP 2010-168294 A (Kao Corp.), 05 August 2010 (05.08.2010), claim 1; paragraphs [0004], [0033] (Family: none) | 1-5 |
| A | JP 2009-62482 A (Kao Corp.), 26 March 2009 (26.03.2009), claims 1, 8; paragraph [0040] (Family: none) | 1-5 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 27 September, 2010 (27.09.10) | 05 October, 2010 (05.10.10) |

| Name and mailing address of the ISA/ | Authorized officer |
|---|---|
| Japanese Patent Office | |
| Facsimile No. | Telephone No. |

Form PCT/ISA/210 (second sheet) (July 2009)

INTERNATIONAL SEARCH REPORT

International application No.
PCT/JP2010/064049

C (Continuation). DOCUMENTS CONSIDERED TO BE RELEVANT

| Category* | Citation of document, with indication, where appropriate, of the relevant passages | Relevant to claim No. |
|---|---|---|
| A | JP 2008-162945 A (Kao Corp.),<br>17 July 2008 (17.07.2008),<br>claim 1; paragraph [0001]<br>(Family: none) | 1-5 |
| A | JP 2006-521302 A (Unilever N.V.),<br>21 September 2006 (21.09.2006),<br>claims 1 to 3, 10, 11; paragraphs [0045], [0046], [0089]<br>& US 2006/0263314 A1 & EP 1605903 A1<br>& WO 2004/084846 A1 | 1-5 |
| A | JP 2004-67668 A (Wella AG.),<br>04 March 2004 (04.03.2004),<br>claims 1, 4, 8; paragraphs [0029], [0031], [0032]<br>& US 2004/0022751 A1 & EP 1386558 A1 | 1-5 |
| A | JP 2005-508917 A (The Procter & Gamble Co.),<br>07 April 2005 (07.04.2005),<br>claim 1; paragraphs [0046], [0065], [0101], [0104]<br>& US 2003/0086886 A1 & EP 1434555 A1<br>& WO 2003/028679 A1 & CN 1564673 A | 1-5 |

Form PCT/ISA/210 (continuation of second sheet) (July 2009)

**INTERNATIONAL SEARCH REPORT**

International application No.
PCT/JP2010/064049

Claim 1 involves hair-dressing cosmetics containing any pressure-sensitive adhesive resins having properties of "being a composition containing a pressure-sensitive adhesive resin substantially at a ratio of 50 mass% and showing a ball number of 1 to 30 in the inclined ball tack test (inclination angle 30°, 25°C, humidity 50%) after forming a film of 0.1 mm in thickness and drying the same for 1 day at 25°C and humidity 50%", sugar alcohol(s) and polyalkylene glycol(s) and one or more kinds of derivatives of the same. However, cosmetics comprising specific pressure-sensitive adhesive resins and specific sugar alcohols or polyalkylene glycols as described in Examples in the description are exclusively disclosed in the meaning within PCT Article 5 and, therefore, the above claim is not supported in the meaning within PCT Article 6.

Thus, the search was made on the scope that is supported by the description.

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2007217314 A
- JP H3261713 A
- JP 2002167317 A
- JP 2004505902 A
- JP HLL100312 A